# EUROPEAN PATENT SPECIFICATION

(11) **EP 3 102 187 B1**
(45) Date of publication and mention of the grant of the patent: **25.03.2020**
(21) Application number: 15702266.6
(22) Date of filing: 03.02.2015
(51) Int. Cl.: A61K 9/20, A61K 31/4985, A61P 3/10

(54) **STABLE PHARMACEUTICAL COMPOSITIONS CONTAINING SITAGLIPTIN IN THE FORM OF IMMEDIATE RELEASE TABLETS**
STABILE PHARMAZEUTISCHE ZUSAMMENSETZUNGEN MIT SITAGLIPTIN IN FORM VON TABLETTEN MIT UNMITTELBARER FREISETZUNG
COMPOSITIONS PHARMACEUTIQUES STABLES CONTENANT DE LA SITAGLIPTINE SE PRÉSENTANT SOUS LA FORME DE COMPRIMÉS À LIBÉRATION IMMÉDIATE

(30) Priority: 03.02.2014 EP 14153671
(43) Date of publication of application: 14.12.2016
(73) Proprietor: Galenicum Health S.L., 08005 Barcelona (ES)
(72) Inventor: ARROYO HIDALGO, Sergio, E-08005 Barcelona (ES); PLADEVALL ROSÉS, MIreia, E-08005 Barcelona (ES)
(74) Representative: Galenicum Health S.L.
(86) International application number: PCT/EP2015/052121
(87) International publication number: WO 2015/114152

(56) References cited:
- EP-A1- 2 578 208
- WO-A1-2005/072530
- WO-A1-2012/131005
- WO-A2-2012/025944
- Jonathan K Reynolds: "Fixed-dose combination of sitagliptin and metformin for the treatment of type 2 diabetes", Diabetes, Diabetes, Metabolic Syndrome and Obesity: Targets and Therapy, 1 July 2009 (2009-07-01), pages 127-134, XP055179538, DOI: http://dx.doi.org/10.2147/DMSO.S4637 Retrieved from the Internet: URL:http://www.dovepress.com/fixed-dose-co mbination-of-sitagliptin-and-metformin-for -the-treatment--peer-reviewed-article-DMSO [retrieved on 2015-03-26]

## Description

The present invention relates to stable pharmaceutical compositions of sitagliptin hydrochloride, in the form of tablets, to a process for the manufacture of said stable pharmaceutical compositions and to uniform pharmaceutical batches of said tablets.

### STATE OF THE ART

Type 2 diabetes is a chronic and progressive disease arising from a complex pathophysiology involving the dual endocrine defects of insulin resistance and impaired insulin secretion. The treatment of type 2 diabetes typically begins with diet and exercise, followed by oral antidiabetic monotherapy. For many patients, these regimens do not sufficiently control glycemia during long-term treatment, leading to a requirement for combination therapy within several years following diagnosis.

Sitagliptin is also known as (*R*)-4-oxo-4-[3-(trifluoromethyl)-5,6-dihydro[1,2,4]triazolo[4,3-*a*]pyrazin-7(8*H*)-yl]-1-(2,4,5-trifluorophenyl)butan-2-amine or MK-0431. The empirical formula of sitagliptin is C16H15F6N5O and the compound has a molecular weight of 407.3 g/mol. The structural formula of sitagliptin is (I):

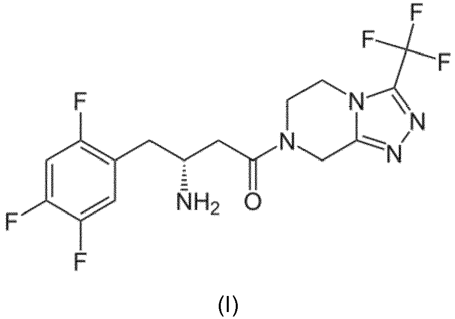

Sitagliptin is a dipeptidyl-peptidase-4 (DPP-4) inhibitor and is used for treatment of diabetes mellitus type 2, also known as non-insulin dependent diabetes mellitus. Sitagliptin is currently marketed in the form of film coated tablets which contain sitagliptin in the form of the phosphate monohydrate salt. Sitagliptin is indicated for adult patients with type 2 diabetes mellitus, to improve glycaemic control. It is indicated as monotherapy in patients inadequately controlled by diet and exercise alone and for whom metformin is inappropriate due to contraindications or intolerance. It is indicated as dual oral therapy in combination with: metformin when diet and exercise plus metformin alone do not provide adequate glycaemic control; a sulphonylurea when diet and exercise plus maximal tolerated dose of a sulphonylurea alone do not provide adequate glycaemic control and when metformin is inappropriate due to contraindications or intolerance, a peroxisome proliferator-activated receptor gamma (PPARγ) agonist (i.e. a thiazolidinedione) when use of a PPARγ agonist is appropriate and when diet and exercise plus the PPARγ agonist alone do not provide adequate glycaemic control. It is indicated as triple oral therapy in combination with a sulphonylurea and metformin when diet and exercise plus dual therapy with these medicinal products do not provide adequate glycaemic control; a PPARγ agonist and metformin when use of a PPARγ agonist is appropriate and when diet and exercise plus dual therapy with these medicinal products do not provide adequate glycaemic control. Sitagliptin is also indicated as add-on to insulin (with or without metformin) when diet and exercise plus stable dose of insulin do not provide adequate glycaemic control.

WO2005/07230A1 relates to crystalline salts of dipeptidyl peptidase-IV inhibitor. More particularly, it relates to crystalline hydrochloric acid, benzenesulfonic acid p-toluenesulfonic acid, 10-camphorsulfonic acid and tartaric salt of (2R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine. According to WO2005/07230A1, the crystalline salts can be formulated into a tablet by a direct compression process.

### DESCRIPTION OF THE INVENTION

The pharmaceutical compositions herein disclosed can be easily manufactured into solid dosage forms, such as tablets, having good stability and the desired dissolution profiles. The pharmaceutical compositions herein disclosed have the technological properties for being manufactured at an industrial scale (flowability, compaction, hardness, disintegration, dissolution and stability).

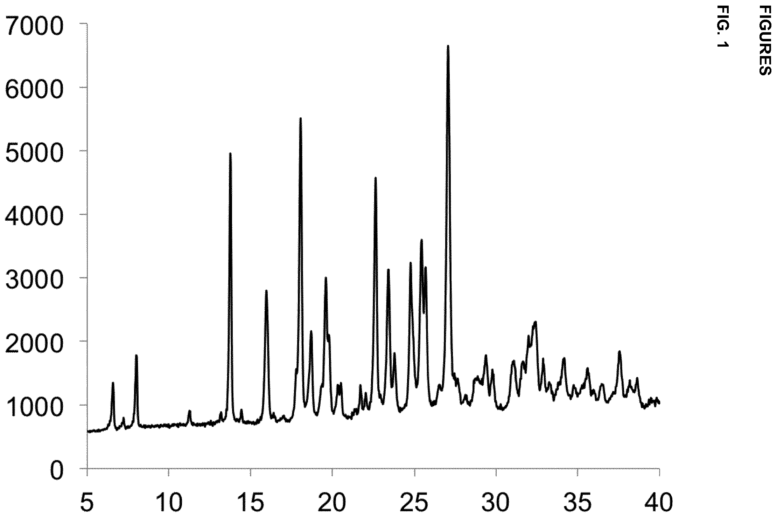

The stable tablets as herein disclosed are uniform in content even when manufactured by dry techniques. The pharmaceutical batches of the pharmaceutical compositions of the present invention have content uniformity. The present invention relates to a pharmaceutical composition in the form of an immediate release tablet comprising sitagliptin hydrochloride or a hydrate thereof and at least one pharmaceutically acceptable excipient prepared by dry granulation. Preferably, the present invention relates to a pharmaceutical composition in the form of an immediate release tablet consisting of sitagliptin hydrochloride or a hydrate thereof and at least one pharmaceutically acceptable excipient. The present invention relates to a pharmaceutical composition comprising at least an active agent, wherein the active agent is selected from: sitagliptin hydrochloride monohydrate; crystalline sitagliptin hydrochloride, preferably the monohydrate; anhydrous sitagliptin hydrochloride; amorphous sitagliptin hydrochloride; and mixtures thereof. In a preferred embodiment, the active agent is sitagliptin hydrochloride monohydrate. In another preferred embodiment, the active agent is crystalline sitagliptin hydrochloride. Preferably, the active agent is crystalline sitagliptin hydrochloride monohydrate. More preferably, the active agent is crystalline sitagliptin hydrochloride monohydrate having at least X-ray powder diffraction peaks 13.8, 18.1 and 27.1° 2θ (±0.2° 2θ). Eve more preferably, the active agent is crystalline sitagliptin hydrochloride monohydrate having at least X-ray powder diffraction peaks 13.8, 18.1, 22.7, 24.8, 25.4 and 27.1° 2θ (±0.2° 2θ). In a preferred embodiment, the active agent is crystalline sitagliptin hydrochloride monohydrate having an X-ray powder diffraction pattern substantially as depicted in Figure 1.

The pharmaceutical compositions as disclosed herein are stable. The term "stable" as used herein refers to a pharmaceutical composition comprising sitagliptin wherein the total content of impurities originated from the decomposition of sitagliptin does not exceed 5 % area, preferably 3 % area, more preferably 2 % area and most preferably 1 % area determined by liquid chromatography (HPLC) at 210 nm if such a composition is stored for at least 2 months at 40°C and 75 % relative humidity (RH).
In a first aspect, the present invention relates to a pharmaceutical composition comprising at least an active agent, wherein the active agent is selected from: sitagliptin hydrochloride monohydrate; crystalline sitagliptin hydrochloride, preferably the monohydrate; anhydrous sitagliptin hydrochloride; amorphous sitagliptin hydrochloride; and mixtures thereof, preferably with the proviso that the pharmaceutical composition does not comprise amorphous sitagliptin, wherein the amorphous sitagliptin has been prepared from a solution comprising sitagliptin and a crystallization inhibitor, wherein the sitagliptin is a sitagliptin salt, wherein the sitagliptin salt is sitagliptin hydrochloride, and wherein the pharmaceutical composition is prepared by dry granulation.

When the active agent is anhydrous sitagliptin hydrochloride, it can be obtained for example by lyophilization from a solution comprising said active agent.

As used herein, the expression "free of crystallisation inhibitor" means that the pharmaceutical composition does not comprises a crystallization inhibitor, or, in case it comprises it, the crystallization inhibitor does not inhibit the crystallisation of the active agent.

In a preferred embodiment, the active agent is sitagliptin hydrochloride monohydrate. In another preferred embodiment, the active agent is crystalline sitagliptin hydrochloride. Preferably, the active agent is crystalline sitagliptin hydrochloride monohydrate. More preferably, the active agent is crystalline sitagliptin hydrochloride monohydrate having at least X-ray powder diffraction peaks 13.8, 18.1 and 27.1° 2θ (±0.2° 2θ). Eve more preferably, the active agent is crystalline sitagliptin hydrochloride monohydrate having at least X-ray powder diffraction peaks 13.8, 18.1, 22.7, 24.8, 25.4 and 27.1° 2θ (±0.2° 2θ). In a preferred embodiment, the active agent is crystalline sitagliptin hydrochloride monohydrate having an X-ray powder diffraction pattern substantially as depicted in Figure 1.

In another preferred embodiment, the sitagliptin hydrochloride is any one of the forms described in WO2010000469 (example 1, pages 9 to 11; example 2, pages 11 and 12), WO2011123641 (pages 18 and 22), WO2012147092 (page 13, example 8 and figure 4), WO2012025944 (page 8, 4th paragraph, pages 25 and 26, examples 10 to 13 and figures 5 to 7) or WO2011025932 (example 5 in page 19 and figure 1 or examples 6 to 8 in pages 20 and 21).

In a preferred embodiment, the active agent is micronized, preferably, the active agent is micronized sitagliptin hydrochloride monohydrate. In a preferred embodiment, the active agent has a particle size volume distribution with a D50 under 40 microns and a D90 under 80 microns, preferably the active agent has a particle size volume distribution with a D50 under 25 microns and a D90 under 60 microns, more preferably, the active agent has a particle size volume distribution with a D50 under 15 microns and a D90 under 20 microns. In a preferred embodiment, the active agent has a particle size volume distribution with a D50 between 1 and 15 microns and a D90 between 3 and 20 microns.

In a preferred embodiment of the first aspect of the present invention, the active agent is amorphous sitagliptin hydrochloride and the pharmaceutical composition is free of a crystallization inhibitor. In another preferred embodiment, the pharmaceutical composition does not comprise amorphous sitagliptin, wherein the amorphous sitagliptin has been prepared from a solution comprising sitagliptin and a crystallization inhibitor, wherein the sitagliptin is a sitagliptin salt, wherein the sitagliptin salt is sitagliptin hydrochloride.

In another preferred embodiment, the active agent is a mixture of sitagliptin hydrochloride monohydrate, crystalline sitagliptin hydrochloride, preferably the monohydrate, and amorphous sitagliptin hydrochloride, and wherein the pharmaceutical composition is free of a crystallization inhibitor.

In a preferred embodiment of the first aspect of the present invention, the pharmaceutical composition is in the form of immediate release tablets, preferably, the pharmaceutical composition is in the form of immediate release film coated tablets. In a preferred embodiment, the active agent is in the tablet core. In a preferred embodiment, the pharmaceutical composition of the present invention is an immediate release film coated tablet with the active agent in the tablet core.

An immediate release tablet as herein disclosed has to be understood as a tablet having a dissolution performance such as 60 % or more of the active agent contained in said pharmaceutical composition dissolves within 60 minutes (min). In a preferred embodiment, the immediate release composition as herein disclosed releases at least 80 % of the active agent in 60 minutes. In another embodiment, the pharmaceutical composition as herein disclosed releases at least 80 % of the active agent in 45 min, preferably in 35 min and more preferably in 30 min. In another embodiment, the pharmaceutical composition as herein disclosed releases at least 80 % of the active agent in 30 min and at least a 95 % of the active agent in 60 min. In a most preferred embodiment, the pharmaceutical composition as herein disclosed releases at least 80 % of the active agent in 15 min, and at least 95 % of the active agent in 60 min. Preferably, the pharmaceutical composition releases at least 85 % of the active agent in 15 min. The dissolution test for an immediate release pharmaceutical composition comprising the active agent as herein disclosed is performed in the following conditions: USP Apparatus: 1 (basket). Speed: 100 rpm. Medium: water. Wavelength: 215 nm.

In a preferred embodiment of the pharmaceutical composition as disclosed herein, sitagliptin is the only active agent. In another preferred embodiment, the pharmaceutical composition further comprises metformin.

In a preferred embodiment of the pharmaceutical composition as disclosed herein, no liquid solvent is used in the preparation of said pharmaceutical composition. As used herein, the term "liquid solvent" refers to any liquid substance in which the active agent can be dissolved. The pharmaceutical composition is prepared by dry granulation. More preferably, the pharmaceutical composition comprises compacted granules containing the active agent. In a preferred embodiment, the compacted granules have a particle size volume distribution with a D90 between 80 and 500 microns, preferably between 100 and 475 microns, more preferably between 150 and 450 microns, when measured by laser diffraction analysis. In another preferred embodiment, the compacted granules have a particle size volume distribution with a D90 between 500 and 800 microns. In another preferred embodiment, the final mix comprising the compacted granules has a particle size volume distribution with a D90 between 500 and 800 microns, before compression of said final mix into tablets

In a preferred embodiment of the pharmaceutical composition as disclosed herein, the weight ratio active agent:total pharmaceutical composition is between 2:1 to 1:20. Preferably, said the weight ratio is between 3:2 to 1:15, more preferably said weight ratio is between 1:1 to 1:10.

In a preferred embodiment of the pharmaceutical composition as disclosed herein, the pharmaceutical composition comprises an amount of active agent per unit dose equivalent to 25 mg, 50 mg or 100 mg of the active agent as free base.

As used herein, the term "unit dose" or "unit dosage" refers to a physically discrete unit that contains a predetermined quantity of active agent calculated to produce a desired therapeutic effect. The unit dose or unit dosage may be in the form of a tablet, capsule, sachet, etc. referred to herein as a "unit dosage form".

In a preferred embodiment, the pharmaceutical composition comprises at least a filler. Preferably, the filler is selected from calcium phosphate dibasic, microcrystalline cellulose, lactose, preferably anhydrous lactose, mannitol, maltose, isomaltose, xylitol, trehalose, starch and mixtures thereof. More preferably, the filler is a mixture of calcium phosphate dibasic and microcrystalline cellulose. When the pharmaceutical composition as herein disclosed comprises excipients such as a filler, a glidant, a lubricant or a disintegrant, these excipients are pharmaceutically acceptable. The term "pharmaceutically acceptable" indicates that the substance or composition must be compatible chemically and/or toxicologically, with the other ingredients comprising a formulation, and/or the mammal being treated therewith. The term "filler" as used herein refers to pharmaceutically acceptable excipients which are added to the bulk volume of the active agent making up the solid composition. As a result, the size of the solid composition increases, which makes its size suitable for handling. Fillers are convenient when the dose of drug per solid composition is low and the solid composition would otherwise be too small.

In a preferred embodiment, the pharmaceutical composition comprises at least a disintegrant. Preferably, the disintegrant is selected from croscarmellose sodium, crospovidone, low-substituted hydroxypropyl cellulose, starch, sodium starch glycolate, carmellose and mixtures thereof. More preferably, the disintegrant is croscarmellose sodium. As used herein, "disintegrant" means a substance or a mixture of substances added to a tablet to facilitate its breakup or disintegration after administration.

In a preferred embodiment, the pharmaceutical composition comprises at least a glidant. Preferably, the glidant is selected from silicon dioxide, magnesium oxide, glyceryl monostearate, glyceryl monooleate, glyceryl behenate and mixtures thereof. More preferably, the glidant is silicon dioxide. As used herein, "glidant" means a substance that improves the flowability of the pharmaceutical composition of the present invention when it is in form of a powder.

In a preferred embodiment, the pharmaceutical composition comprises at least a lubricant. Preferably, the lubricant is selected from sodium stearyl fumarate, magnesium stearate, calcium stearate talc, stearic acid and mixtures thereof. More preferably, the lubricant is sodium stearyl fumarate, magnesium stearate or a mixture thereof, preferably is a mixture thereof. As used herein, "lubricant" means a substance that reduces friction between the composition of the present invention and the surfaces of the apparatus used to compact the composition into a compressed form.

In a preferred embodiment, the water content of the pharmaceutical composition is below 7 % by weight in respect of the total amount of the pharmaceutical composition. Preferably, the water content is between 0.5 % and 5 % by weight in respect of the total amount of the pharmaceutical composition.

In a preferred embodiment of the pharmaceutical composition as disclosed herein, the active agent is sitagliptin hydrochloride monohydrate, the pharmaceutical composition is in the form of immediate release tablets, the active agent is in the tablet core, which comprises compacted granules having a particle size volume distribution with a D90 between 150 and 450 microns, when measured by laser diffraction analysis. In a preferred embodiment of the pharmaceutical composition as disclosed herein, the active agent is sitagliptin hydrochloride monohydrate, the pharmaceutical composition is in the form of immediate release tablets, the active agent is in the tablet core, which consists essentially of compacted granules having a particle size volume distribution with a D90 between 150 and 450 microns, when measured by laser diffraction analysis.

In a preferred embodiment of the pharmaceutical composition as disclosed herein, the active agent is crystalline sitagliptin hydrochloride monohydrate having an X-ray powder diffraction pattern substantially as depicted in Figure 1, the pharmaceutical composition is prepared by dry granulation and comprises an amount of active agent per unit dose equivalent to 25 mg, 50 mg or 100 mg of the active agent as free base.

In another preferred embodiment of the pharmaceutical composition as disclosed herein, the pharmaceutical composition is in the form of an immediate release tablet, the active agent is selected from: sitagliptin hydrochloride monohydrate; crystalline sitagliptin hydrochloride, preferably the monohydrate; amorphous sitagliptin hydrochloride; and mixtures thereof, (provided that the pharmaceutical composition comprising amorphous sitagliptin hydrochloride is free of a crystallization inhibitor), and wherein the pharmaceutical composition comprises a core and a film coating, wherein the core comprises calcium phosphate dibasic, microcrystalline cellulose, croscarmellose sodium, silicon dioxide, sodium stearyl fumarate and magnesium stearate. In another preferred embodiment of the pharmaceutical composition as disclosed herein, the pharmaceutical composition is in the form of an immediate release tablet, the active agent is selected from: sitagliptin hydrochloride monohydrate; crystalline sitagliptin hydrochloride, preferably the monohydrate; amorphous sitagliptin hydrochloride; and mixtures thereof, (provided that the pharmaceutical composition comprising amorphous sitagliptin hydrochloride is free of a crystallization inhibitor), and wherein the pharmaceutical composition consists of a core and a film coating, wherein the core consists essentially of, preferably consists of the active agent, calcium phosphate dibasic, microcrystalline cellulose, croscarmellose sodium, silicon dioxide, sodium stearyl fumarate and magnesium stearate.

In another preferred embodiment of the pharmaceutical composition as disclosed herein, the pharmaceutical composition consists essentially of crystalline sitagliptin hydrochloride having a particle size volume distribution with a D50 under 15 microns and a D90 under 20 microns and at least one pharmaceutically acceptable excipient, wherein the pharmaceutical composition is prepared by dry granulation.

In another aspect, the present invention relates to a pharmaceutical granulate comprising at least an active agent, wherein the active agent is selected from: sitagliptin hydrochloride monohydrate; crystalline sitagliptin hydrochloride, preferably the monohydrate; anhydrous sitagliptin hydrochloride; amorphous sitagliptin hydrochloride; and mixtures thereof, preferably with the proviso that the pharmaceutical composition does not comprise amorphous sitagliptin, wherein the amorphous sitagliptin has been prepared from a solution comprising sitagliptin and a crystallization inhibitor, wherein the sitagliptin is a sitagliptin salt, wherein the sitagliptin salt is sitagliptin hydrochloride.

As used herein, the term "granulate" refers to a population of granules. The granules as disclosed herein, can be prepared by any granulation method. As used herein, the term "granulation" refers to the process of agglomerating powder particles into larger agglomerates (i.e. granules) that contain the active agent. The term "granulation" includes dry and wet granulation techniques. The term "wet granulation" refers to any process comprising the steps of addition of a liquid to the powder starting materials, preferably kneading, and drying to yield a solid dosage form. The term "dry granulation" refers to any process that comprises compacting the powder, usually either by slugging or with a roller compactor, and preferably milling the compacted powder to obtain the granules. No liquid is employed for the dry granulation. The compacted granulate or compacted granules as disclosed herein are prepared by dry granulation.

In a preferred embodiment, the granulate is made by compaction and milling. In another preferred embodiment, the granulate has a particle size volume distribution with a D90 between 80 and 500 microns, preferably between 100 and 475 microns, more preferably between 150 and 450 microns, when measured by laser diffraction analysis.

In another aspect, the present invention relates to an isolated micronized crystalline sitagliptin hydrochloride, preferably an isolated micronized crystalline sitagliptin hydrochloride monohydrate. In a preferred embodiment, the isolated micronized crystalline sitagliptin hydrochloride monohydrate has at least X-ray powder diffraction peaks 13.8, 18.1 and 27.1° 2θ (±0.2° 2θ). Preferably, it has at least X-ray powder diffraction peaks 13.8, 18.1, 22.7, 24.8, 25.4 and 27.1° 2θ (±0.2° 2θ). Also, the present invention relates to the use of said micronized crystalline sitagliptin hydrochloride, preferably an isolated micronized crystalline sitagliptin hydrochloride monohydrate, for the manufacture of a pharmaceutical composition of the first aspect of the present invention. As used herein, the term "micronization" refers to a decrease in particle size through application of force to a particle, resulting in the break-up of the particle. Such force may be applied by collision of particles at high speeds.

In another aspect, the present invention relates to the pharmaceutical composition of the first aspect, for use to improve glycaemic control in the treatment of diabetes. Preferably, for use in the treatment of type 2 diabetes mellitus. More preferably, for use in the treatment of type 2 diabetes mellitus alone or in combination with other antidiabetic agents.

In another aspect, the present invention relates to a pharmaceutical batch comprising at least 20,000 units, preferably at least 50,000 units, more preferable at least 100,000 units of the pharmaceutical composition of the first aspect. In a preferred embodiment, the content of the active agent is uniform. In a preferred embodiment, the pharmaceutical compositions are packaged in a blister pack of aluminum/PVC, aluminum/aluminum or PVC/PE/PVDC/aluminum.

In another aspect, the present invention relates to the pharmaceutical batch of the present invention for use in the treatment of type 2 diabetes mellitus.

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

The pharmaceutical composition may be manufactured at laboratory scale, not necessarily following GMP and not intended for commercialization. The pharmaceutical composition may also be manufactured for validation, following GMP. A batch of a pharmaceutical composition which is manufactured for validation is called "pilot batch".

Each pharmaceutical batch of finished product must fulfil the regulatory requirements of the corresponding Medicine Agency before being released for sale or supply, such as impurities thresholds and stability data.

The term "uniform" as used herein refers to the content of the active agent in the tablets of a pharmaceutical batch has to be homogeneous. According to the FDA criteria, uniformity is considered as achieving 90-110 % potency of the theoretical strength with a relative standard deviation (RSD) of less than 5 % for all samples (Guidance for Industry ANDA's: Blend Uniformity Analysis, published August 1999).

The term "active agent" refers to a therapeutically active compound, as well as any prodrugs thereof and pharmaceutically acceptable salts, hydrates and solvates of the compound and the prodrugs.

For the release of a pharmaceutical batch the distribution of the active agent in the tablets has to be homogeneous, that is, content uniformity is required. All batches are expected to be uniform within normal process variation. Process validation studies are conducted prior to the marketing of a drug product to assure that production processes are controlled. The test batch is manufactured prior to validation, yet it is the basis on which an application is approved (MANUAL OF POLICIES AND PROCEDURES, MAPP 5225.1).

It is therefore essential to assure that the test batch is uniform. In-process tests for uniformity should be conducted throughout the entire production process, e.g., at commencement or completion of significant phases (21 CFR 211.110). These tests should be designed to detect potential in-process anomalies (MAPP 5225.1).

In another aspect, the present invention relates to the use of sitagliptin hydrochloride or a hydrate thereof for the preparation of a pharmaceutical composition comprising sitagliptin hydrochloride or a hydrate thereof. Preferably, the sitagliptin hydrochloride hydrate is the monohydrate. More preferably, the sitagliptin hydrochloride is crystalline sitagliptin hydrochloride monohydrate having an X-ray powder diffraction pattern substantially as depicted in Figure 1. In another preferred embodiment, the pharmaceutical composition is in the form of immediate release tablets comprising a tablet core which comprises compacted granules containing the sitagliptin hydrochloride or a hydrate thereof.

In another aspect, the present invention relates to a process for the manufacture of a pharmaceutical composition of the first aspect, wherein sitagliptin free base is not used in the manufacture of the pharmaceutical composition.

In another aspect, the present invention relates to a process for the manufacture of a pharmaceutical composition of the first aspect, wherein isolated sitagliptin hydrochloride is used in the manufacturing process of the pharmaceutical composition.

In another aspect, the present invention relates to a process for the manufacture of a pharmaceutical composition comprising sitagliptin hydrochloride or a hydrate thereof, comprising the following steps:
(i) weighing, sieving and mixing the active agent and optionally at least one pharmaceutically acceptable excipient, and
(ii) compressing the mix obtained in step (i) to form a tablet.

In another aspect, the present invention relates to a process for the manufacture of a pharmaceutical composition comprising sitagliptin hydrochloride or a hydrate thereof, comprising the following steps:
(i) weighing, sieving and mixing the active agent and optionally at least one pharmaceutically acceptable excipient, and
(ii) compacting the mix obtained in step (i),
(iii) granulating the compacted mix obtained in step (ii),
(iv) optionally mixing the granules obtained in step (iii) with at least one pharmaceutically acceptable excipient, and
(v) compressing the granules obtained in step (iii) or the mix obtained in step (iv) to form a tablet.

In another aspect, the present invention relates to a process for the manufacture of the pharmaceutical composition comprising at least an active agent, wherein the active agent is selected from: sitagliptin hydrochloride monohydrate; crystalline sitagliptin hydrochloride, preferably the monohydrate; anhydrous sitagliptin hydrochloride; amorphous sitagliptin hydrochloride; and mixtures thereof, provided that the pharmaceutical composition comprising amorphous sitagliptin hydrochloride is free of a crystallization inhibitor, comprising the following steps:
(i) weighing, sieving and mixing the active agent and optionally at least one pharmaceutically acceptable excipient, and
(ii) compressing the mix obtained in step (i) to form a tablet.

In another aspect, the present invention relates to a process for the manufacture of the pharmaceutical composition comprising at least an active agent, wherein the active agent is selected from: sitagliptin hydrochloride monohydrate; crystalline sitagliptin hydrochloride, preferably the monohydrate; anhydrous sitagliptin hydrochloride; amorphous sitagliptin hydrochloride; and mixtures thereof, provided that the pharmaceutical composition comprising amorphous sitagliptin hydrochloride is free of a crystallization inhibitor, comprising the following steps:
(i) weighing, sieving and mixing the active agent and optionally at least one pharmaceutically acceptable excipient,
(ii) granulating the mix of step (i),
(iii) optionally mixing the granules of step (ii) with at least one pharmaceutically acceptable excipient, and
(iv) compressing the granules obtained in step (ii) or the mix obtained in step (iii) to form a tablet.

In another aspect, the present invention relates to a process for the manufacture of the pharmaceutical composition comprising at least an active agent, wherein the active agent is selected from: sitagliptin hydrochloride monohydrate; crystalline sitagliptin hydrochloride, preferably the monohydrate; anhydrous sitagliptin hydrochloride; amorphous sitagliptin hydrochloride; and mixtures thereof, provided that the pharmaceutical composition comprising amorphous sitagliptin hydrochloride is free of a crystallization inhibitor, comprising the following steps:
(i) weighing, sieving and mixing the active agent and optionally at least one pharmaceutically acceptable excipient, and
(ii) compacting the mix obtained in step (i),
(iii) granulating the compacted mix obtained in step (ii),
(iv) optionally mixing the granules obtained in step (iii) with at least one pharmaceutically acceptable excipient, and
(v) compressing the granules obtained in step (iii) or the mix obtained in step (iv) to form a tablet.

In another aspect, the present invention relates to the pharmaceutical composition manufactured according to the processes as disclosed herein. Another aspect of the present invention relates to the pharmaceutical composition of the previous aspect for use in the treatment of type 2 diabetes mellitus.

In another aspect, the present invention relates to a process for the manufacture of a pharmaceutical composition of the first aspect, in the form of an immediate release tablet, wherein the process comprises the following steps:
(i) preparing a test pharmaceutical composition in the form of a tablet comprising an active agent selected from: sitagliptin hydrochloride monohydrate; crystalline sitagliptin hydrochloride, preferably the monohydrate; amorphous sitagliptin hydrochloride; and mixtures thereof, provided that the pharmaceutical composition comprising amorphous sitagliptin hydrochloride is free of a crystallization inhibitor;
(ii) checking the stability and dissolution profile of the test pharmaceutical composition of step (i); and
(iii) manufacturing a pharmaceutical composition in the form of a tablet comprising an active agent selected from: sitagliptin hydrochloride monohydrate; crystalline sitagliptin hydrochloride, preferably the monohydrate; amorphous sitagliptin hydrochloride; and mixtures thereof, provided that the pharmaceutical composition comprising amorphous sitagliptin hydrochloride is free of a crystallization inhibitor, in the form of an immediate release tablet by the same process used to prepare the test pharmaceutical composition in step (i) provided that the test composition is stable and has an immediate release dissolution profile; and
(iv) optionally, packaging the stable pharmaceutical composition manufactured in step (iii), preferably in a blister pack or a bottle.

The term "blister" or bubble pack refers to a sheet in a package construction with recesses designed to hold dosage forms. The sheet may be a plastic, a foil, or combination thereof. Normally, a blister is a product consisting of a flat structure in which blisters are formed, generally by means of a heating process, into which the single elements to be packaged are inserted. The blisters are then hermetically sealed using flat strips of appropriate thermoformable materials (plastics, aluminum, paper), which represent the frangible element through which it is then possible to remove the product. The primary component of a blister pack is a cavity or pocket made from a formable web, usually a thermoformed plastic. This usually has a backing of paperboard or a lidding seal of aluminum foil or plastic. An aluminum/PVC blister refers to a blister where the thermoformable material is from PVC and the backing is a lidding seal of aluminum foil. A PVC/PE/PVDC/aluminum blister refers to a blister where the thermoformable material is a laminate of PVC/PE/PVDC and the backing is a lidding seal of aluminum foil. PVC is poly(vinylchloride), PE is polyethylene and PVDC is polyvinylidene chloride. Blister packs are commonly used as unit-dose packaging for pharmaceutical tablets, capsules or lozenges. Blister packs can provide barrier protection for shelf life requirements, and a degree of tamper resistance. In the USA, blister packs are mainly used for packing physician samples of drug products, or for Over The Counter (OTC) products in the pharmacy. In other parts of the world, blister packs are the main packaging type since pharmacy dispensing and re-packaging are not common. A series of blister cavities is sometimes called a blister card or blister strip as well as blister pack. The difference between a strip pack and blister pack is that a strip pack does not have thermo-formed or cold formed cavities; the strip pack is formed around the tablet at a time when it is dropped to the sealing area between sealing moulds. In some parts of the world the pharmaceutical blister pack is known as a Push-Through-Pack (PTP), an accurate description of two key properties (i) the lidding foil is brittle allowing to press the product out while breaking the lidding foil and (ii) a semi-rigid formed cavity being sufficiently collapsable to be able to dispense the tablet or capsule by means of pressing it out with your thumb. The main advantages of unit-dose blister packs over other methods of packing pharmaceutical products are the assurance of product/packaging integrity (including shelflife) of each individual dose and the possibility to create a compliance pack or calendar pack by printing the days of the week above each dose. Blister packs can be created by means of a form-fill-seal process at the pharmaceutical company or designated contract packer. A form-fill-seal process means that the blister pack is created from rolls of flat sheet or film, filled with the pharmaceutical product and closed (sealed) on the same equipment. Such equipment is called a blisterline. There are two types of blister machine's design: rotary and flat-plate.

In another aspect, the present invention relates to a process for the manufacture of a pharmaceutical batch of a pharmaceutical composition of the first aspect, in the form of an immediate release tablet, wherein the process comprises the following steps:
(i) preparing a test pharmaceutical composition of sitagliptin hydrochloride monohydrate; crystalline sitagliptin hydrochloride, preferably the monohydrate; amorphous sitagliptin hydrochloride; or mixtures thereof, provided that the pharmaceutical composition comprising amorphous sitagliptin hydrochloride is free of a crystallization inhibitor, in the form of a tablet;
(ii) checking the stability and dissolution profile of the test pharmaceutical composition of step (i); and
(iii) manufacturing a pharmaceutical batch by the same manufacturing process used to prepare the test pharmaceutical composition in step (i) provided that the test composition is stable and has an immediate release dissolution profile; and
(iv) optionally, packaging the pharmaceutical batch manufactured in step (iii) preferably in blister packs or in bottles.

In a preferred embodiment, the stability checked in step (ii) is the stability after at least one day at 40° C and 75 % relative humidity.

In another aspect, the present invention relates to a process for the validation of a pharmaceutical batch as disclosed herein, comprising the following steps:
(i) manufacturing the pharmaceutical batch;
(ii) checking the uniformity of the active agent content; and
(iii) validating the batch only if the content is uniform.

In another aspect, the present invention relates to a blister pack comprising the pharmaceutical composition of the first aspect or the pharmaceutical composition manufactured according to the processes as disclosed herein, wherein said blister pack is preferably an aluminum/PVC blister, an aluminum/aluminum blister or a PVC/PE/PVDC/aluminum blister. Preferably, said blister pack is a PVC/PE/PVDC/aluminum blister.

In another aspect, the present invention relates to a cardboard box with a patient information leaflet comprising at least one aluminum/aluminum or aluminum/PVC or PVC/PE/PVDC/aluminum blister pack of at least 4 units of the pharmaceutical composition of the first aspect or the pharmaceutical composition manufactured according to the processes as disclosed herein.

In another aspect, the present invention relates to a cardboard box with a patient information leaflet according to the preceding claim comprising at least one aluminum/PVC or PVC/PE/PVDC/aluminum blister pack of at least 4 units of the pharmaceutical composition of the first aspect or the pharmaceutical composition manufactured according to the processes as disclosed herein.

In another aspect, the present invention relates to a cardboard box with a patient information leaflet comprising a bottle containing at least 10 units of the pharmaceutical composition of the first aspect or the pharmaceutical composition manufactured according to the processes as disclosed herein.

All percentages, parts, and ratios herein are by weight unless specifically noted otherwise. As used herein, the term "about" refers preferably to a range that is ± 10 %, preferably ± 5 %, or more preferably ± 1 % of a value with which the term is associated.

### DESCRIPTION OF THE FIGURES

FIG. 1: X-ray powder diffraction pattern (XRD) of crystalline sitagliptin hydrochloride monohydrate.

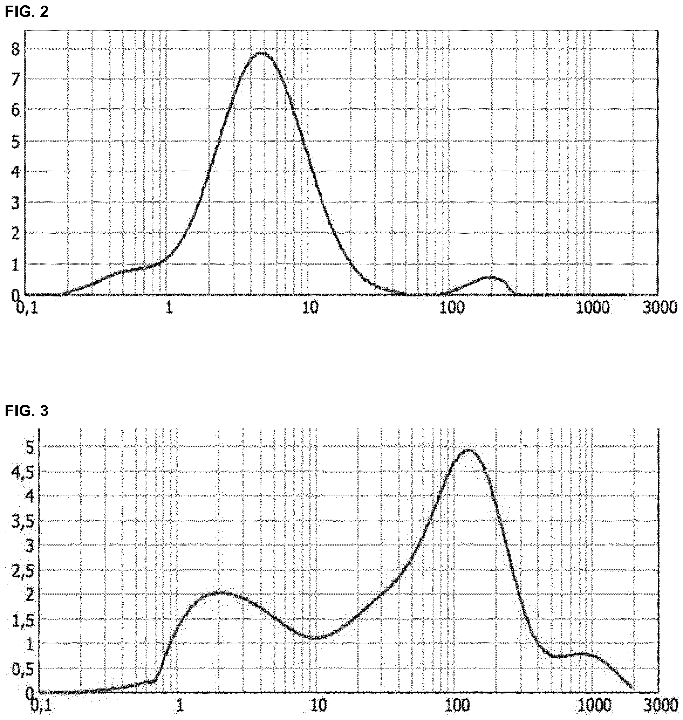

FIG. 2: Particle size volume distribution of sitagliptin hydrochloride monohydrate. Horizontal axis: particle size in micrometers. Vertical axis: volume (%), indicating the percentage of particles with the corresponding particle size.
FIG. 3: Particle size volume distribution of the granules comprising sitagliptin hydrochloride monohydrate. Horizontal axis: particle size in micrometers. Vertical axis: volume (%), indicating the percentage of particles with the corresponding particle size.

### EXAMPLES

### Examples 1 to 3: sitagliptin compositions

| Examples | 1 | 2 | 3 |
|---|---|---|---|
| sitagliptin hydrochloride monohydrate (mg) | 27.24* | 54.48** | 108.95*** |
| Calcium Phosphate Dibasic (mg) | 31.88 | 63.76 | 127.53 |
| Microcrystalline cellulose (mg) | 31.88 | 63.76 | 127.53 |
| Croscarmellose sodium (mg) | 5.00 | 10.00 | 20.00 |
| Silicon dioxide (mg) | 1.00 | 2.00 | 4.00 |
| Sodium stearyl fumarate (mg) | 2.00 | 4.00 | 8.00 |
| Magnesium Stearate (mg) | 1.00 | 2.00 | 4.00 |
| Total (mg) | 100.00 | 200.00 | 400.00 |

| | | | |
|---|---|---|---|
| * Equivalent to 25 mg sitagliptin free base. ** Equivalent to 50 mg sitagliptin free base. *** Equivalent to 100 mg sitagliptin free base. | | | |

### Example 4: Batches of compositions 1, 2 and 3 manufacture by dry granulation

All ingredients were sifted through a 0.5 mm sieve. The sitagliptin hydrochloride was mixed with all of the excipients except for the lubricant during 15 min at 34 rpm. The dry mix was then compacted either by slugging or with a roller compactor. The compacted mix was milled and sieved through a 2.36 mm sieve. The lubricant was added and mixed with the compacted granulate during 5 min at 34 rpm. The lubricated granulate was compressed into tablets. Batches of 500 to 5,000 tablets were manufactured for the compositions of examples 1, 2 and 3. These batches are referred to as batches 1, 2 and 3.

### Examples 5 to 7: sitagliptin compositions

| Examples | 5 | 6 | 7 |
|---|---|---|---|
| sitagliptin hydrochloride monohydrate (mg) | 27.24* | 54.48** | 108.95*** |
| Calcium Phosphate Dibasic (mg) | 31.63 | 63.26 | 126.53 |
| Microcrystalline cellulose (mg) | 31.63 | 63.26 | 126.53 |
| Croscarmellose sodium (mg) | 5.00 | 10.00 | 20.00 |
| Silicon dioxide (mg) | 1.00 | 2.00 | 4.00 |
| Sodium stearyl fumarate (mg) | 2.50 | 5.00 | 10.00 |
| Magnesium Stearate (mg) | 1.00 | 2.00 | 4.00 |
| Total (mg) | 100.00 | 200.00 | 400.00 |

| | | | |
|---|---|---|---|
| * Equivalent to 25 mg sitagliptin free base. ** Equivalent to 50 mg sitagliptin free base. *** Equivalent to 100 mg sitagliptin free base. | | | |

### Example 8: Batches of compositions 5, 6 and 7 manufacture by dry granulation

All ingredients were sifted through a 0.5 mm sieve. The sitagliptin hydrochloride was mixed with all of the excipients except for the lubricant. The dry mix was then compacted either by slugging or with a roller compactor. The compacted mix was milled and sieved. The lubricant was added and mixed with the compacted granulate. The lubricated granulate was compressed into tablets. Batches of 35,000 to 140,000 tablets were manufactured for the compositions of examples 5, 6 and 7. These batches are referred to as batches 5, 6 and 7.

### Example 9: Content uniformity

The sitagliptin content and the amount of impurities were analysed by HPLC. The area corresponding to the sitagliptin and of the major peaks, if any, was determined. The percentage of impurities was calculated by comparing the areas of the measured peaks with those obtained from the standard.

### Example 10: Stability

Tablets of examples 1, 2 and 3 and 5, 6 and 7 were packaged in blisters packs of aluminum/PVC or aluminum/aluminum or PVC/PE/PVDC/aluminum. Tablets of examples 1, 2 and 3 and 5, 6 and 7 were subjected to stability tests at times 0, 15 days, 1 month (1 m), 2 months (2 m), 3 months (3 m) and 6 months (6 m) at 25° C and 60 % Relative Humidity (RH), at 30° C and 65 % RH, and at 40° C and 75 % RH. The total impurities for batch 7 are shown below.

| Impurity % | Ex. 7 / 1 m | Ex. 7 / 2 m | Ex. 7 / 3 m | Ex. 7 / 6 m |
|---|---|---|---|---|
| 25°C/60%RH | < 0.05 | < 0.05 | < 0.05 | 0.13 |
| 30°C/65%RH | < 0.05 | < 0.05 | < 0.05 | 0.13 |
| 40°C/75%RH | < 0.05 | < 0.05 | < 0.05 | 0.26 |

### Example 7: Water content

The amount of water of the pharmaceutical compositions as herein disclosed was measured by loss on drying (LOD, 105° C) using a Halogen Moisture Analyzer. The water content of batches 1, 2 and 3 and 5, 6 and 7 was very similar, and ranged from 3 to 4 %.

### Example 8: Particle size volume distribution

The particle size distribution of the active agent was analysed by laser diffraction spectroscopy using a Malvern Mastersizer 2000 particle size analyzer.

The particle size distribution of the granulate was also analysed by laser diffraction spectroscopy using a Malvern Mastersizer 2000 particle size analyzer.

### Example 9: Synthesis of sitagliptin hydrochloride monohydrate

The process for the synthesis of sitagliptin is described in EP1412357. The process of synthesis of sitagliptin hydrochloride monohydrate is described in WO2005072530, example 1 in page 15. The sitagliptin hydrochloride monohydrate obtained by this process was characterised by X-Ray Powder Diffraction and is crystalline the form depicted in Fig. 1. Also, crystalline sitagliptin hydrochloride can be prepared as described in WO2010000469 (example 1, pages 9 to 11; example 2, pages 11 and 12), WO2011123641 (pages 18 and 22), WO2012147092 (page 13, example 8 and figure 4), WO2012025944 (page 8, 4th paragraph, pages 25 and 26, examples 10 to 13 and figures 5 to 7) or WO2011025932 (example 5 in page 19 and figure 1 or examples 6 to 8 in pages 20 and 21).

### Example 10: X-Ray Powder Diffraction

X-Ray Powder Diffraction was performed in a Bruker D8 Advance diffractometer with a θ:2θ configuration and Bragg-Brentano geometry with a copper anode tube. The diffractogram is obtained for 2θ angles ranging from 3° to 70° with a step of 0.03° each second. The tube set-up is 40 kV and 30 mA, incident-beam divergence-limiting slit 12 mm, static sample, diffracted-beam receiving slit 0.2 mm and Nickel filter.

Crystalline sitagliptin hydrochloride monohydrate of figure 1 has the following peaks:

| Angle 2θ | Intensity |
|---|---|
| 6.6 | 1,342 |
| 8.0 | 1,775 |
| 13.8 | 4,948 |
| 16.0 | 2,797 |
| 18.1 | 5,511 |
| 19.6 | 3,001 |
| 22.7 | 4,574 |
| 23.4 | 3,130 |
| 24.8 | 3,239 |
| 25.4 | 3,576 |
| 25.7 | 3,165 |
| 27.1 | 6,647 |

Unless otherwise indicated, all the analysis methods are carried out according to the European Pharmacopoeia 7th edition.

## Claims

1. A pharmaceutical composition in the form of immediate release tablets comprising at least an active agent, wherein the active agent is crystalline sitagliptin hydrochloride, preferably the monohydrate, wherein the pharmaceutical composition is prepared by dry granulation.

2. The pharmaceutical composition according to claim 1, with the proviso that the pharmaceutical composition does not comprise amorphous sitagliptin, wherein the amorphous sitagliptin has been prepared from a solution comprising sitagliptin and a crystallization inhibitor, wherein the sitagliptin is a sitagliptin salt, wherein the sitagliptin salt is sitagliptin hydrochloride.

3. The pharmaceutical composition according to the preceding claim, wherein the active agent is crystalline sitagliptin hydrochloride monohydrate having at least X-ray powder diffraction peaks 13.8, 18.1 and 27.1° 2θ (±0.2° 2θ); preferably, the active agent is crystalline sitagliptin hydrochloride monohydrate having an X-ray powder diffraction pattern substantially as depicted in Figure 1; or wherein the active agent is micronized; or wherein the active agent has a particle size volume distribution with a D50 under 40 micrometers (microns) and a D90 under 80 micrometers (microns); preferably, the active agent has a particle size volume distribution with a D50 under 15 micrometers (microns) and a D90 under 20 micrometers (microns); more preferably, the active agent has a particle size volume distribution with a D50 between 1 and 15 micrometers (microns) and a D90 between 3 and 20 micrometers (microns).

4. The pharmaceutical composition according to any one of the preceding claims, wherein said pharmaceutical composition is in the form of immediate release film coated tablets, and/or wherein the active agent is in the tablet core.

5. The pharmaceutical composition according to any one of the preceding claims, wherein sitagliptin is the only active agent or wherein the pharmaceutical composition further comprises metformin.

6. The pharmaceutical composition according to any one of the preceding claims, wherein no liquid solvent is used in the preparation of said pharmaceutical composition; and/or wherein said pharmaceutical composition comprises compacted granules containing the active agent, preferably the compacted granules have a particle size volume distribution with a D90 between 80 and 500 micrometers (microns), when measured by laser diffraction analysis, more preferably, the compacted granules have a particle size volume distribution with a D90 between 150 and 450 micrometers (microns), when measured by laser diffraction analysis.

7. The pharmaceutical composition according to any one of the preceding claims, wherein the weight ratio active agent:total pharmaceutical composition is between 2:1 to 1:20, preferably the weight ratio active agent:total pharmaceutical composition is between 1:1 to 1:10; and/or wherein the pharmaceutical composition comprises an amount of active agent per unit dose equivalent to 25 mg, 50 mg or 100 mg of the active agent as free base.

8. The pharmaceutical composition according to any one of the preceding claims, wherein the pharmaceutical composition comprises at least a filler, preferably the filler is a mixture of calcium phosphate dibasic and microcrystalline cellulose; and/or wherein the pharmaceutical composition comprises at least a disintegrant, preferably the disintegrant is croscarmellose sodium; and/or wherein the pharmaceutical composition comprises at least a glidant, preferably the glidant is silicon dioxide; and/or wherein the pharmaceutical composition comprises at least a lubricant, preferably the lubricant is sodium stearyl fumarate, magnesium stearate or a mixture thereof, preferably is a mixture thereof.

9. The pharmaceutical composition according to any one of the preceding claims, wherein the water content is below 7 % by weight in respect of the total amount of the pharmaceutical composition, preferably the water content is between 0.5 % and 5 % by weight in respect of the total amount of the pharmaceutical composition.

10. Isolated micronized crystalline sitagliptin hydrochloride, preferably micronized crystalline sitagliptin hydrochloride monohydrate, more preferably micronized crystalline sitagliptin hydrochloride monohydrate, for use in the manufacture of a pharmaceutical composition according to claim 1.

11. A compacted granulate comprising at least an active agent, wherein the active agent is selected from crystalline sitagliptin hydrochloride, preferably the monohydrate, wherein the compacted granulate has a particle size volume distribution with a D90 between 80 and 500 micrometers (microns), when measured by laser diffraction analysis, more preferably, the compacted granulate has a particle size volume distribution with a D90 between 150 and 450 micrometers (microns), when measured by laser diffraction analysis, wherein the compacted granulate is prepared by dry granulation.

12. A uniform pharmaceutical batch comprising at least 50,000 units, preferably comprising at least 500,000 units of the pharmaceutical composition as defined in any one of claims 1 to 8 , preferably wherein the content of the active agent is uniform, preferably wherein the pharmaceutical compositions are packaged in a blister pack of aluminum/PVC, aluminum/aluminum or PVC/PE/PVDC/aluminum.

13. A process for the manufacture of the pharmaceutical composition according to claims 1 to 8 comprising at least an active agent, wherein the active agent is crystalline sitagliptin hydrochloride, preferably the monohydrate, comprising the following steps:
(i) weighing, sieving and mixing the active agent and optionally at least one pharmaceutically acceptable excipient, and
(ii) compacting the mix obtained in step (i),
(iii) optionally granulating the compacted mix obtained in step (ii),
(iv) optionally mixing the granules obtained in step (iii) with at least one pharmaceutically acceptable excipient, and
(v) compressing the granules obtained in step (iii) or the mix obtained in step (iv) to form a tablet.

14. The pharmaceutical composition according to any one of claims 1 to 8, or the pharmaceutical batch according to claim 12, for use in the treatment of type 2 diabetes mellitus.

15. A packaged pharmaceutical composition wherein said packaged pharmaceutical composition is: a blister pack comprising the pharmaceutical composition as defined in any one of claims 1 to 7 or in claim 13, or a cardboard box with a patient information leaflet comprising at least one blister pack of at least 4 units of the pharmaceutical composition as defined in any one of claims 1 to 7 or in claim 13, or a cardboard box with a patient information leaflet comprising a bottle containing at least 10 units of the pharmaceutical composition as defined in any one of claims 1 to 8 or in claim 13.

16. A process for the manufacture of the pharmaceutical composition comprising at least an active agent, wherein the active agent is selected from: crystalline sitagliptin hydrochloride, preferably the monohydrate; comprising the following steps:
(i) weighing, sieving and mixing the active agent, wherein the said active agent has a particle size volume distribution with a D50 under 15 micrometers (microns) and a D90 under 20 micrometers (microns); more preferably, said active agent has a particle size volume distribution with a D50 between 1 and 15 micrometers (microns) and a D90 between 3 and 20 micrometers (microns), and optionally at least one pharmaceutically acceptable excipient;
(ii) granulating the mix of step (i), wherein the provided granulate has a particle size volume distribution with a D90 between 80 and 500 micrometers (microns), more preferably, having a particle size volume distribution with a D90 between 150 and 450 micrometers (microns), when measured by laser diffraction analysis;
(iii) optionally mixing the granules of step (ii) with at least one pharmaceutically acceptable excipient; and
(iv) compressing the granules obtained in step (ii) or the mix obtained in step (iii) to form a tablet.

17. A pharmaceutical composition obtainable by the process defined in claim 16.

## Patentansprüche

1. Pharmazeutische Zusammensetzung in Form von Tabletten mit sofortiger Freisetzung, umfassend mindestens einen Wirkstoff, wobei der Wirkstoff kristallines Sitagliptin Hydrochlorid, vorzugsweise das Monohydrat, ist, wobei die pharmazeutische Zusammensetzung durch Trockengranulation hergestellt wird.

2. Pharmazeutische Zusammensetzung nach Anspruch 1, mit der Maßgabe, dass die pharmazeutische Zusammensetzung kein amorphes Sitagliptin umfasst, wobei das amorphe Sitagliptin aus einer Lösung hergestellt wurde, die Sitagliptin und einen Kristallisationshemmer umfasst, wobei das Sitagliptin ein Sitagliptinsalz ist, wobei das Sitagliptinsalz Sitagliptin-Hydrochlorid ist.

3. Pharmazeutische Zusammensetzung nach dem vorhergehenden Anspruch, wobei der Wirkstoff kristallines Sitagliptin Hydrochlorid Monohydrat mit mindestens den Röntgenpulverbeugungs-Peaks 13,8, 18,1 und 27,1° 2θ (± 0,2° 2θ) ist; wobei vorzugsweise der Wirkstoff kristallines Sitagliptin Hydrochlorid Monohydrat mit einem Röntgenpulverbeugungsmuster ist, das im wesentlichen wie in Figur 1 dargestellt ist; oder wobei der Wirkstoff mikronisiert ist; oder wobei der Wirkstoff eine Teilchengrößen-Volumenverteilung mit einem D50 unter 40 Mikrometern (Mikron) und einem D90 unter 80 Mikrometern (Mikron) aufweist; vorzugsweise der Wirkstoff eine Teilchengrößen-Volumenverteilung mit einem D50 unter 15 Mikrometern (Mikron) und einem D90 unter 20 Mikrometern (Mikron) aufweist; wobei der Wirkstoff mehr bevorzugt eine Teilchengrößen-Volumenverteilung mit einem D50 zwischen 1 und 15 Mikrometern (Mikron) und einem D90 zwischen 3 und 20 Mikrometern (Mikron) aufweist.

4. Pharmazeutische Zusammensetzung nach einem der vorhergehenden Ansprüche, wobei die pharmazeutische Zusammensetzung in Form von Filmtabletten mit sofortiger Freisetzung vorliegt und/oder wobei der Wirkstoff in dem Tablettenkern vorliegt.

5. Pharmazeutische Zusammensetzung nach einem der vorhergehenden Ansprüche, wobei Sitagliptin der einzige Wirkstoff ist oder wobei die pharmazeutische Zusammensetzung ferner Metformin umfasst.

6. Pharmazeutische Zusammensetzung nach einem der vorhergehenden Ansprüche, wobei bei der Herstellung der pharmazeutischen Zusammensetzung kein flüssiges Lösungsmittel verwendet wird; und/oder wobei die pharmazeutische Zusammensetzung verdichtete Granulate umfasst, die den Wirkstoff enthalten, wobei die verdichteten Granulate vorzugsweise eine Teilchengrößen-Volumenverteilung mit einem D90 zwischen 80 und 500 Mikrometern (Mikron) aufweisen, wenn sie durch Laserbeugungsanalyse gemessen werden, wobei die verdichteten Granulate mehr bevorzugt eine Teilchengrößen-Volumenverteilung mit einem D90 zwischen 150 und 450 Mikrometern (Mikron) aufweisen, wenn sie durch Laserbeugungsanalyse gemessen werden.

7. Pharmazeutische Zusammensetzung nach einem der vorhergehenden Ansprüche, wobei das Gewichtsverhältnis Wirkstoff:gesamte pharmazeutische Zusammensetzung zwischen 2:1 und 1:20 liegt, vorzugsweise das Gewichtsverhältnis Wirkstoff: gesamte pharmazeutische Zusammensetzung zwischen 1:1 und 1:10 liegt; und/oder wobei die pharmazeutische Zusammensetzung eine Menge an Wirkstoff pro Einzeldosis umfasst, die 25 mg, 50 mg oder 100 mg Wirkstoff als freier Base entspricht.

8. Pharmazeutische Zusammensetzung nach einem der vorhergehenden Ansprüche, wobei die pharmazeutische Zusammensetzung mindestens einen Füllstoff umfasst, wobei der Füllstoff vorzugsweise ein Gemisch aus zweibasigem Calciumphosphat und mikrokristalliner Cellulose ist; und/oder wobei die pharmazeutische Zusammensetzung mindestens ein Sprengmittel umfasst, wobei das Sprengmittel vorzugsweise Croscarmellose-Natrium ist; und/oder wobei die pharmazeutische Zusammensetzung mindestens ein Fließmittel umfasst, wobei das Fließmittel vorzugsweise Siliciumdioxid ist; und/oder wobei die pharmazeutische Zusammensetzung mindestens ein Gleitmittel umfasst, wobei das Gleitmittel vorzugsweise Natriumstearylfumarat, Magnesiumstearat oder ein Gemisch davon ist, vorzugsweise ein Gemisch davon ist.

9. Pharmazeutische Zusammensetzung nach einem der vorhergehenden Ansprüche, wobei der Wassergehalt unter 7 Gew.-%, bezogen auf die Gesamtmenge der pharmazeutischen Zusammensetzung, liegt, vorzugsweise der Wassergehalt zwischen 0,5 Gew.-% und 5 Gew.-%, bezogen auf die Gesamtmenge der pharmazeutischen Zusammensetzung liegt.

10. Isoliertes mikronisiertes kristallines Sitagliptin Hydrochlorid, vorzugsweise mikronisiertes kristallines Sitagliptin Hydrochlorid Monohydrat, mehr bevorzugt mikronisiertes kristallines Sitagliptin Hydrochlorid Monohydrat, zur Verwendung bei der Herstellung einer pharmazeutischen Zusammensetzung nach Anspruch 1.

11. Verdichtetes Granulat, umfassend mindestens einen Wirkstoff, wobei der Wirkstoff aus kristallinem Sitagliptin Hydrochlorid, vorzugsweise dem Monohydrat, ausgewählt ist, wobei das verdichtete Granulat eine Teilchengrößen-Volumenverteilung mit einem D90 zwischen 80 und 500 Mikrometern (Mikron) aufweist, wenn es durch Laserbeugungsanalyse gemessen wird, wobei das verdichtete Granulat mehr bevorzugt eine Teilchengrößen-Volumenverteilung mit einem D90 zwischen 150 und 450 Mikrometern (Mikron) aufweist, wenn es durch Laserbeugungsanalyse gemessen wird, wobei das verdichtete Granulat durch Trockengranulation hergestellt wird.

12. Einheitliche pharmazeutische Charge, umfassend mindestens 50 000 Einheiten, vorzugsweise umfassend mindestens 500 000 Einheiten der pharmazeutischen Zusammensetzung nach einem der Ansprüche 1 bis 8, wobei der Gehalt des Wirkstoffs vorzugsweise einheitlich ist, wobei die pharmazeutischen Zusammensetzungen vorzugsweise in einer Blisterpackung aus Aluminium/PVC, Aluminium/Aluminium oder PVC/PE/PVDC/Aluminium verpackt sind.

13. Verfahren zur Herstellung der pharmazeutischen Zusammensetzung nach Anspruch 1 bis 8, umfassend mindestens einen Wirkstoff, wobei der Wirkstoff kristallines Sitagliptin Hydrochlorid, vorzugsweise das Monohydrat, ist, umfassend die folgenden Schritte:
(i) Wiegen, Sieben und Mischen des Wirkstoffs und gegebenenfalls mindestens eines pharmazeutisch annehmbaren Hilfsstoffs und
(ii) Verdichten des in Schritt (i) erhaltenen Gemisches,
(iii) gegebenenfalls Granulieren des in Schritt (ii) erhaltenen verdichteten Gemisches,
(iv) gegebenenfalls Mischen der in Schritt (iii) erhaltenen Granulate mit mindestens einem pharmazeutisch annehmbaren Hilfsstoff und
(v) Verpressen des in Schritt (iii) erhaltenen Granulats oder des in Schritt (iv) erhaltenen Gemisches, um eine Tablette zu bilden.

14. Pharmazeutische Zusammensetzung nach einem der Ansprüche 1 bis 8 oder pharmazeutische Charge nach Anspruch 12 zur Verwendung bei der Behandlung von Typ-2-Diabetes mellitus.

15. Verpackte pharmazeutische Zusammensetzung, wobei die verpackte pharmazeutische Zusammensetzung Folgendes ist: eine Blisterpackung, umfassend die pharmazeutische Zusammensetzung nach einem der Ansprüche 1 bis 7 oder nach Anspruch 13 oder ein Karton mit einem Beipackzettel, umfassend mindestens eine Blisterpackung von mindestens 4 Einheiten der pharmazeutischen Zusammensetzung nach einem der Ansprüche 1 bis 7 oder nach Anspruch 13 oder ein Karton mit einem Beipackzettel, umfassend eine Flasche, die mindestens 10 Einheiten der pharmazeutischen Zusammensetzung nach einem der Ansprüche 1 bis 8 oder nach Anspruch 13 enthält.

16. Verfahren zur Herstellung der pharmazeutischen Zusammensetzung, umfassend mindestens einen Wirkstoff, wobei der Wirkstoff ausgewählt ist aus: kristallinem Sitagliptin Hydrochlorid, vorzugsweise dem Monohydrat, umfassend die folgenden Schritte:
(i) Wiegen, Sieben und Mischen des Wirkstoffs, wobei der Wirkstoff eine Teilchengrößen-Volumenverteilung mit einem D50 unter 15 Mikrometern (Mikron) und einem D90 unter 20 Mikrometern (Mikron) aufweist; wobei der Wirkstoff mehr bevorzugt eine Teilchengrößen-Volumenverteilung mit einem D50 zwischen 1 und 15 Mikrometern (Mikron) und einem D90 zwischen 3 und 20 Mikrometern (Mikron) und gegebenenfalls mindestens einen pharmazeutisch annehmbaren Hilfsstoff aufweist;
(ii) Granulieren des Gemisches aus Schritt (i), wobei das bereitgestellte Granulat eine Teilchengrößen-Volumenverteilung mit einem D90 zwischen 80 und 500 Mikrometern (Mikron), mehr bevorzugt eine Teilchengrößen-Volumenverteilung mit einem D90 zwischen 150 und 450 Mikrometern (Mikron) aufweist, wenn es durch Laserbeugungsanalyse gemessen wird;
(iii) gegebenenfalls Mischen der Granulate aus Schritt (ii) mit mindestens einem pharmazeutisch annehmbaren Hilfsstoff und
(iv) Verpressen des in Schritt (ii) erhaltenen Granulats oder des in Schritt (ill) erhaltenen Gemisches, um eine Tablette zu bilden.

17. Pharmazeutische Zusammensetzung, welche durch das Verfahren nach Anspruch 16 erhältlich ist.

## Revendications

1. Composition pharmaceutique sous forme de comprimés à libération immédiate comprenant au moins un agent actif, dans laquelle l'agent actif est le chlorhydrate de sitagliptine cristallin, de préférence le monohydrate, dans laquelle la composition pharmaceutique est préparée par granulation à sec.

2. Composition pharmaceutique selon la revendication 1, à condition que la composition pharmaceutique ne comprenne pas de sitagliptine amorphe, dans laquelle la sitagliptine amorphe a été préparée à partir d'une solution comprenant de la sitagliptine et un inhibiteur de cristallisation, dans laquelle la sitagliptine est un sel de sitagliptine, dans laquelle le sel de sitagliptine est le chlorhydrate de sitagliptine.

3. Composition pharmaceutique selon la revendication précédente, dans laquelle l'agent actif est le chlorhydrate de sitagliptine cristallin monohydraté ayant au moins les pics de diffraction des rayons X sur poudre 13,8, 18,1 et 27,1 ° 2θ (± 0,2 ° 2θ); de préférence, l'agent actif est le chlorhydrate de sitagliptine cristallin monohydraté ayant un diagramme de diffraction des rayons X sur poudre, sensiblement tel que représenté sur la figure 1 ; ou dans laquelle l'agent actif est micronisé ; ou dans laquelle l'agent actif a une distribution en volume granulométrique avec un D50 inférieur à 40 micromètres (microns) et un D90 inférieur à 80 micromètres (microns) ; de préférence, l'agent actif a une distribution en volume granulométrique avec un D50 inférieur à 15 micromètres (microns) et un D90 inférieur à 20 micromètres (microns) ; plus préférablement, l'agent actif a une distribution en volume granulométrique avec un D50 compris entre 1 et 15 micromètres (microns) et un D90 compris entre 3 et 20 micromètres (microns).

4. Composition pharmaceutique selon l'une quelconque des revendications précédentes, dans laquelle ladite composition pharmaceutique est sous la forme de comprimés pelliculés à libération immédiate, et/ou dans laquelle l'agent actif est dans le noyau du comprimé.

5. Composition pharmaceutique selon l'une quelconque des revendications précédentes, dans laquelle la sitagliptine est le seul agent actif ou dans laquelle la composition pharmaceutique comprend en outre de la metformine.

6. Composition pharmaceutique selon l'une quelconque des revendications précédentes, dans laquelle aucun solvant liquide n'est utilisé dans la préparation de ladite composition pharmaceutique ; et/ou dans laquelle ladite composition pharmaceutique comprend des granulés compactés contenant l'agent actif, de préférence, les granulés compactés ont une distribution en volume granulométrique avec un D90 compris entre 80 et 500 micromètres (microns), lorsqu'ils sont mesurés par analyse par diffraction laser, plus préférablement, les granulés compactés ont une distribution en volume granulométrique avec un D90 compris entre 150 et 450 micromètres (microns), lorsqu'ils sont mesurés par analyse par diffraction laser.

7. Composition pharmaceutique selon l'une quelconque des revendications précédentes, dans laquelle le rapport pondéral agent actif: composition pharmaceutique totale est compris entre 2:1 et 1:20, de préférence, le rapport pondéral agent actif: composition pharmaceutique totale est compris entre 1:1 et 1:10; et/ou dans laquelle la composition pharmaceutique comprend une quantité d'agent actif par dose unitaire équivalente à 25 mg, 50 mg ou 100 mg de l'agent actif sous forme de base libre.

8. Composition pharmaceutique selon l'une quelconque des revendications précédentes, dans laquelle la composition pharmaceutique comprend au moins une charge, de préférence, la charge est un mélange de phosphate de calcium dibasique et de cellulose microcristalline ; et/ou dans laquelle la composition pharmaceutique comprend au moins un agent désintégrant, de préférence, l'agent désintégrant est la croscarmellose sodique ; et/ou dans laquelle la composition pharmaceutique comprend au moins un agent glissant, de préférence, l'agent glissant est le dioxyde de silicium ; et/ou dans laquelle la composition pharmaceutique comprend au moins un lubrifiant, de préférence le lubrifiant est le stéarylfumarate de sodium, le stéarate de magnésium ou un mélange de ces derniers, est de préférence un mélange de ces derniers.

9. Composition pharmaceutique selon l'une quelconque des revendications précédentes, dans laquelle la teneur en eau est inférieure à 7 % en poids par rapport à la quantité totale de la composition pharmaceutique, de préférence, la teneur en eau est comprise entre 0,5 % et 5 % en poids par rapport à la quantité totale de la composition pharmaceutique.

10. Chlorhydrate de sitagliptine cristallin micronisé isolé, de préférence le chlorhydrate de sitagliptine cristallin micronisé monohydraté, plus préférablement, le chlorhydrate de sitagliptine cristallin micronisé monohydraté, en vue de son utilisation dans la fabrication d'une composition pharmaceutique selon la revendication 1.

11. Granulat compacté comprenant au moins un agent actif, dans lequel l'agent actif est choisi parmi le chlorhydrate de sitagliptine cristallin, de préférence le monohydrate, dans lequel le granulat compacté a une distribution en volume granulométrique avec un D90 compris entre 80 et 500 micromètres (microns), lorsqu'ils sont mesurés par analyse par diffraction laser, plus préférablement, le granulat compacté a une distribution en volume granulométrique avec un D90 compris entre 150 et 450 micromètres (microns), lorsqu'ils sont mesurés par analyse par diffraction laser, dans laquelle le granulat compacté est préparé par granulation à sec.

12. Lot pharmaceutique homogène comprenant au moins 50 000 unités, comprenant de préférence au moins 500 000 unités de la composition pharmaceutique telle que définie dans l'une quelconque des revendications 1 à 8, de préférence dans lequel le contenu de l'agent actif est homogène, de préférence dans lequel les compositions pharmaceutiques sont conditionnées dans un blister en aluminium/PVC, aluminium/aluminium ou PVC/PE/PVDC/aluminium.

13. Procédé de fabrication de la composition pharmaceutique selon les revendications 1 à 8 comprenant au moins un agent actif, dans laquelle l'agent actif est le chlorhydrate de sitagliptine cristallin, de préférence le monohydrate, comprenant les étapes suivantes consistant à :
(i) peser, tamiser et mélanger l'agent actif et éventuellement au moins un excipient pharmaceutiquement acceptable, et
(ii) compacter le mélange obtenu à l'étape (i),
(iii) granuler éventuellement le mélange compacté obtenu à l'étape (ii),
(iv) mélanger éventuellement les granulés obtenus à l'étape (iii) avec au moins un excipient pharmaceutiquement acceptable, et
(v) comprimer les granulés obtenus à l'étape (iii) ou le mélange obtenu à l'étape (iv) pour former un comprimé.

14. Composition pharmaceutique selon l'une quelconque des revendications 1 à 8, ou le lot pharmaceutique selon la revendication 12, en vue de son utilisation dans le traitement du diabète de type 2.

15. Composition pharmaceutique conditionnée dans laquelle ladite composition pharmaceutique conditionnée est : un blister comprenant la composition pharmaceutique telle que définie dans l'une quelconque des revendications 1 à 7 ou dans la revendication 13, ou une boîte en carton avec une notice d'information pour le patient comprenant au moins un blister d'au moins 4 unités de la composition pharmaceutique telle que définie dans l'une quelconque des revendications 1 à 7 ou dans la revendication 13, ou une boîte en carton avec une notice d'information pour le patient comprenant un flacon contenant au moins 10 unités de la composition pharmaceutique telle que définie dans l'une quelconque des revendications 1 à 8 ou dans la revendication 13.

16. Procédé de fabrication de la composition pharmaceutique comprenant au moins un agent actif, dans lequel l'agent actif est choisi parmi : le chlorhydrate de sitagliptine cristallin, de préférence le monohydrate ; comprenant les étapes suivantes consistant à :
(i) peser, tamiser et mélanger l'agent actif, dans lequel ledit agent actif a une distribution en volume granulométrique avec un D50 inférieur à 15 micromètres (microns) et un D90 inférieur à 20 micromètres (microns) ; plus préférablement, ledit agent actif a une distribution en volume granulométrique avec un D50 compris entre 1 et 15 micromètres (microns) et un D90 compris entre 3 et 20 micromètres (microns), et éventuellement au moins un excipient pharmaceutiquement acceptable ;
(ii) granuler le mélange de l'étape (i), dans lequel le granulat fourni a une distribution en volume granulométrique avec un D90 compris entre 80 et 500 micromètres (microns), plus préférablement, ayant une distribution en volume granulométrique avec un D90 compris entre 150 et 450 micromètres (microns), lorsqu'ils sont mesurés par analyse par diffraction laser ;
(iii) mélanger éventuellement les granulés de l'étape (ii) avec au moins un excipient pharmaceutiquement acceptable ; et
(iv) comprimer les granulés obtenus à l'étape (ii) ou le mélange obtenu à l'étape (iii) pour former un comprimé.

17. Composition pharmaceutique pouvant être obtenue par le procédé défini dans la revendication 16.
